# EUROPEAN PATENT APPLICATION

(11) **EP 3 067 051 A1**
(43) Date of publication of application: **14.09.2016**
(21) Application number: 16166429.7
(22) Date of filing: 17.02.2012
(51) Int. Cl.: A61K 31/19, C07C 409/24, A61K 31/327, A61K 31/22, A01N 37/16

(54) **COMPOSITIONS COMPRISING PEROXY ALPHA-KETOCARBOXYLIC ACID AND METHODS FOR PRODUCING AND USING THE SAME**

(30) Priority: 17.02.2011 US 201161444111 P; 02.12.2011 US 201161565986 P
(62) Divisional of application: 12747111.8
(71) Applicant: CHD Bioscience, Inc., Fort Collins, CO 80523 (US)
(72) Inventor: Neas, Edwin D., Nunn, CO Colorado 80648 (US); Skinner, John D., Fort Collins, CO Colorado 80525 (US)
(74) Representative: Andrews, Timothy Stephen

(57) **Abstract**

There is provided a composition comprising a peroxy α-ketocarboxylic acid, which contains an acidic -OOH group, and at least one of a carboxylic acid corresponding to the peroxy α-ketocarboxylic acid and an anion of the carboxylic acid corresponding to the peroxy α-ketocarboxylic acid, wherein the acidic -OOH group of the α-ketocarboxylic acid is replaced by a -OH group in the carboxylic acid corresponding to the peroxy α-ketocarboxylic acid.

## Description

### CROSS-REFERENCE TO RELATED APPLICATIONS

This application claims the priority benefit of U.S. Provisional Application Nos. 61/444,111, filed February 17, 2012, and 61/565,986, filed December 2, 2011, all of which are incorporated herein by reference in their entirety.

### FIELD OF THE INVENTION

The present invention relates to compositions comprising peroxy α-ketocarboxylic acid and methods for using and producing the same. In some particular embodiments, compositions of the invention also include α-ketoesters.

### BACKGROUND OF THE INVENTION

The skin is the body's largest organ and serves as the primary protective barrier to the outside world. Any physical disruption (i.e., wound) to this organ must therefore be quickly and efficiently repaired in order to restore tissue integrity and function. Quite often proper wound healing is impaired with devastating consequences such as severe morbidity, amputations, or death. In humans and animals, protection from mechanical injury, chemical hazards, and bacterial invasion is provided by the skin because the epidermis is relatively thick and covered with keratin. Secretions from sebaceous glands and sweat glands also benefit this protective barrier. In the event of an injury that damages the skin's protective barrier, the body triggers a response called wound healing.

The classical model of wound healing is divided generally into four sequential, yet overlapping, phases: (1) hemostasis, (2) inflammatory, (3) proliferative and (4) remodeling. The hemostasis phase involves platelets (thomboctytes) to form a fibrin clot to control active bleeding. The inflammatory phase involves migration of phagocytes to the wound to kill microorganisms and release of subsequent signaling factors to involve the migration and division of cells involved in the proliferative phase. The proliferative phase involves vascular cell production for angiogenesis, fibroblast cells to excrete collagen and fibronectin to form an extracellular matrix, and epithelial cells to reform the external epidermis. In addition, the wound is made smaller by myofibroblasts. Finally, collagen is remodeled and cells that are no longer needed are removed by programmed cell death (i.e., apoptosis).

The process of wound healing can be divided into two major phases: early phase and cellular phase. The early phase includes hemostasis that involves vasoconstriction, temporary blockage of a break by a platelet plug, and blood coagulation, or formation of a clot that seals the hole until tissues are repaired. The early phase also includes the generation of stimuli to attract the cellular responses needed to instigate inflammation. In the inflammation phase, white blood cells, or leukocytes, are attracted to the wound site by platelet-derived growth factor (PDGF), and these cells of the immune system are involved in defending the body against both infectious disease and foreign materials.

Currently, there are 18 other known proteins involved in the inflammatory phase which interact to regulate this response. For example, IL-4, IL-10, and IL-13 are potent activators of B lymphocytes. However, IL-4, IL-10, and IL-13 are also potent anti-inflammatory agents. The phagocytic cells engulf and then digest cellular debris and pathogens and stimulate lymphocytes and other immune cells to respond to the wound area. Once the invading microorganisms have been brought under control, the skin proceeds through the proliferative and remodeling stage by a complex cascade of biochemical events orchestrated to repair the damage. This involves the formation of a scab within several hours. The scab temporarily restores the integrity of the epidermis and restricts the entry of microorganisms. After the scab is formed, cells of the stratum basale begin to divide by mitosis and migrate to the edges of the scab. A week after the injury, the edges of the wound are pulled together by contraction. Contraction is an important part of the healing process when damage has been extensive, and involves shrinking in size of underlying contractile connective tissue, which brings the wound margins toward one another. In a major injury, if epithelial cell migration and tissue contraction cannot cover the wound, suturing the edges of the injured skin together, or even replacement of lost skin with skin grafts, may be required to restore the skin. Interruption of this healing process by a breakdown in any of these wound healing processes will lead to a chronic wound. Depending on the severity of the wound, the proliferative phase and final maturation of the wound to complete scar tissue can take from days up to years.

The molecular events in the wound healing process of acute, chronic and burn wounds continue to be studied. It has been found that wound healing exhibits an extremely complex array of biochemical events involving a regulated cascade of inter and intra cellular events. One of the rapidly growing fields in wound healing research is based on cellular growth factors and the use of these factors for the treatment of wounds. The biochemical response at the cellular level is a process involving intricate interactions among different cell functions that include energy production, structural proteins, growth factors, and proteinases. The treatment of wounds with known cellular growth factors has a potential to help heal wounds by stimulating the cellular processes involved in angiogenesis, cellular proliferation, regulating the production and degradation of the extracellular matrix, and attracting the inflammatory cells and fibroblasts to the wound. While many biochemical reactions involving wound healing has been discovered, the entire process of wound healing is not fully understood at this point.

Currently, many wound treatment protocols involve the use of molecular stimulators such as nucleotides, polysaccharides, and/or proteins (generally referred to as growth factors), and antioxidants. These cellular molecules function to incite cellular, matrix, angiogenesis and other response(s) within the wound to enhance the healing process. Since there are numerous metabolic events that occur during wound healing processes, it is generally believed that none of the conventional wound healing methods are all encompassing solution to efficient and safe wound healing. Some of the limitations for many of conventional wound healing treatments are inability to efficiently deliver some of these compounds to deep wound cells involved in wound healing, inability to address the problem of infection control with sanitizers and/or antibiotics, and/or cost justification for affordable treatment plans and competition with anti-inflammatory medications.

Other skin wounds involve burns. Major burns are relatively common injuries that require multidisciplinary treatment for patient survival and recovery. It is estimated that more than 30,000 people die each year worldwide because of fire-related burn injuries. Many more are seriously injured, disabled, or disfigured because of burn injuries. There have been significant advances in medical care for burns over the last 15 years due to fluid resuscitation, wound cleaning, skin replacement, infection control, and nutritional support. These changes have primarily resulted from the use of early burn wound excursion, early adequate nutrition, and the use of surgical techniques that minimize blood and heat loss. Since modern treatment of burns has greatly advanced, sepsis has become the leading cause of death after a burn injury. Multiple antibiotic resistant bacteria now account for the bulk of deaths due to sepsis in burn victims, the etiology of which is believed to be due to antibiotic resistant bacteria and biofilm formation in the wound and extraneous nosocomial infections. It has been estimated that there is a 75% mortality rate in older burn patients due to sepsis resulting from *Aspergillus niger* infection. The common antiseptic treatment for burns, silver sulfanide, will not kill these spores in the burn wound, and therefore currently there is no effective treatment for this problem.

Impediments to wound healing include hypoxia, infection, presence of debris and necrotic tissue, use of inflammatory medications, a diet deficient in vitamins or minerals or general nutrition, tumors, environmental factors, and metabolic disorders such as diabetes mellitus. It is believed that the primary impediments to healing an acute wound are hypoxia, infection, wound debris, and/or anti-inflammatory medications. Typical standard of care for wounds generally involves wound debridement, dressing and administration of antibiotics, if infection occurs.

Despite many advances in wound treatment, there is a continuing need for new composition for treating wounds. And with rising cases of drug resistant sepsis infection, there is an urgent need for a composition that can effectively treat drug resistant sepsis infection.

### SUMMARY OF THE INVENTION

Some aspects of the invention provide compositions comprising a mixture of an α-ketoester and a peroxy α-ketocarboxylic acid (PKCA). In some embodiments, compositions of the invention also include an α-ketoacid. Within these embodiments, in some instances said α-ketoacid is a decarboxylated α-ketoacid of said PKCA. In other embodiments, said α-ketoester comprises an alkyl α-ketoester. Within these embodiments, in some instances said alkyl α-ketoester is an alkyl pyruvate ester. Yet in other embodiments, the molar ratio of said α-ketoester to said PKCA is from about 0.02:1 to about 10:1. Still in other embodiments, said PKCA comprises peroxy α-ketopyruvic acid, peroxy α-ketobutyric acid, peroxy α-ketovaleric acid, or a mixture thereof. Yet in other embodiments, said composition is formulate as a gel, a liquid, lotion, skin patch, irrigation gel, a spray, or a combination thereof.

Other aspects of the invention provide methods for reducing the amount of microbe on a surface. Such methods typically comprise contacting the surface with a composition comprising an effective amount of a mixture of an α-ketoester and a peroxy α-ketocarboxylic acid. In some embodiments, the microbe comprises vegetative bacteria. In other embodiments, the microbe comprises bacterial spores, mycobacteria, gram-negative bacteria, vegetative gram-positive bacteria, or a combination thereof.

Yet other aspects of the invention provide methods for reducing the number of infectious vegetative bacteria on a substrate. Such methods generally include contacting the substrate with a composition comprising an effective amount of a mixture of an α-ketoester and a peroxy α-ketocarboxylic acid.

Still other aspects of the invention provide methods for preventing and/or reducing bacteria-related diseases in a mammal that result from the mammal's contact with a bacteria-infected substrate. Such methods comprise contacting the substrate with a composition comprising an effective amount of a mixture of an α-ketoester and a peroxy α-ketocarboxylic acid.

In other aspects of the invention provide methods for treating wound in a subject. Such methods comprise topically administering a composition comprising a peroxy α-ketocarboxylic acid to the wound area of the subject. In some embodiments, the composition further comprises an α-ketoester.

Still yet other aspects of the invention provide methods for preventing sepsis from a wound in a subject. Such methods comprise topically administering a composition comprising an effective amount of a peroxy α-ketocarboxylic acid to the wound of the subject. In some embodiments, the composition further comprises an α-ketoester.

### BRIEF DESCRIPTION OF THE DRAWINGS

Figure 1 is a graph showing efficacy of PKCA compounds against *Clostridium difficile* spores.
Figure 2 is a picture showing the results of various concentrations of PPA treatment on biofilm formation.
Figure 3 is a picture showing the results of various concentrations of PPA-EP treatment on biofilm.
Figure 4 is a picture showing the results of various concentrations of PPA and PPA-EP on eliminating formed biofilm.
Figure5 is a graph showing the results PPA efficacy at different concentrations against MRSA in FBS.
Figure 6 is a graph showing the results of PPA efficacy at different concentrations against *MRSA* in PBS.
Figure 7 is a graph showing the results of PPA efficacy at different concentrations against *A baumannii* in PBS.
Figure 8 is a graph showing the results of PPA efficacy at different concentrations of *Pseudomonas* in egg yolk.
Figure 9 shows a composition of the invention that is formulated in a variety of different sized dissolvable film.
Figure 10 shows the blood agar plate that was treated with different concentrations of PPA dissolvable films.

### DETAILED DESCRIPTION OF THE INVENTION

### The Wound Healing Antagonist: Infection

### Traumatic Wound Infections

Open wounds that are healing naturally are often contaminated by skin flora such as coagulase-negative *staphylococci.* The distribution and density of the flora is dependent on a variety of factors including, but not limited to, age and environmental factors such as temperature and humidity, which typically changes with the geographical area. Wounds resulting from trauma is often contaminated with the skin micro flora and the environmental micro flora present on the surfaces where the trauma occurs. Although not universal, a microbial load of ≥ 10⁵ bacteria per gram of tissue is considered an infection. Below these microbial levels, the term "colonization" is used to describe the presence of non-replicating bacteria on a wound surface that does not initiate a significant host immune response.

Wound infection is generally defined as the invasion and multiplication of microorganisms in a wound resulting in tissue injury and illiciting a host immune reaction. Without being bound by any theory, it is generally believed that when the microbial population in the wound exceeds 10⁵, the presence of microorganism stimulates a significant host immune response in the form of a strong inflammatory response phase in the wound healing process. If a gross infection is not treated early and there are multi-drug resistant organisms (MDRO) within the wound, complications of the inflammatory immune response can occur, such as sepsis, subsequent morbidity, a chronic wound with subsequent amputation, or even mortality.

Again without being bound by any theory, it is believed that some of the factors leading to a complication in wound healing due to infection include, but are not limited to, prior or present history of antibiotic use, an infected in-dwelling intravenous catheter, previous history of an antibiotic resistant bacterial infection, an impaired or compromised immune system, and a continual open wound. Currently, the most prevalent pathogens involved in skin and soft tissue infections are believed to be *Staphylococcus aureus* (e.g., methicillin-resistant *Staphylococcus aureus* or MRSA), *Enterococcus sp, coagulase-negative staphylocccus* species, *Escherichia coli,* and *Pseudomonas aeruginosa.* Most of these bacteria are multidrug resistant organisms (MDRO). The fungal spore *Aspergillus sp.,* which is resistant to the current therapeutic treatment, is believed to be the leading cause of sepsis and death after burn injuries. *Staphylococcus aureus* and group A *streptococcus* species are considered the pathogens most involved in infections of the skin outside of hospital settings. Wound infections often lead to long term care with significant costs to the patient, their family, and the medical treatment facilities.

### Chronic Wound Infections

It has been estimated that 1-2% of the populations in Denmark and the United States have a non-healing wound. The predominant microorganisms involved in chronic wound infections include various faculative anaerobes such as *Staphylococcus*, *Corynebacterium, Pseudomonas*, *Serratia, Bacteroides* and the anaerobes *Prevotella*, *Peptostreptococcus*, and *Porphyromonas*. In some cases, microorganisms form a biofilm, i.e., an aggregate of microorganisms in which cells adhere to each other on a surface. Two of the primary biofilm forming infectious organisms are *Staphylococcus aureus* and *Pseudomonas aeruginosa.* Bacteria living in biofilms are very well protected against antibiotics and other antimicrobial agents. Besides avoiding biocide eradication, biofilm forming bacteria, such as *Pseudomonas aeruginosa* can evade the body's defense mechanism by the up regulating synthesis of molecules that can eliminate host defense cells such as polymorphonuclear neutrophilic leukocytes (PMNs).

Bacteria living in biofilms are very well protected against antibiotics and other antimicrobial agents. Typically a wound is considered to be chronic if the wound has not shown 20%-40% reduction in area after 4 weeks. Some define a chronic wound as those that have not healed in 3 months. Microbial biofilm formation in wounds is now well documented. There are many bacteria (as high as 95 species) within the wound that progress into producing a mature biofilm with a protective matrix and continued maturity to enhance survival against antimicrobial treatment methods including topical antibiotic treatments. An existing wound, when ineffectively treated, may progress into a chronic wound, which may continue to grow in size and severity.

In-hospital delay of elective surgery or long term hospital care after surgery has been associated with increase in infectious complications and mortality. In recent years, there has been a dramatic increase in instants of nosocomial bacterial infections in hospitals. It is estimated that nosocomial infections following surgical procedures or incidental wounds occur greater than 5,000 per hospital per year. A health care cost for such nosocomial infections is estimated to be nearly $100,000.00 per case and increasing. It is believed that these infections are primarily due to wound patient's exposure to other contaminated patient, contaminated surgical room surfaces, contaminated medical devices, and/or hand carriage by health care workers, patients and visitors.

As stated above, it is believed that the most problematic microbes in the health care facilities are the antibiotic resistant bacterium, such as Methicillin-Resistant *Staphylococcus aureus* (MRSA), Vancomycin Resistant *Enterococci* (VRE), *Acinetobacter baumannii,* and bacterial spores such as *Clostridium difficile.* It is believed that *Clostridium difficile* can persist for many months in Hospital environments and the vegetative form can be induced to the spore form with certain germicides such as detergents and hypochlorites. Hospital acquired infections from these particular microbes have increased patient cost by approximately 60% over 20 years and raised mortality rates from 5.7 per million to 23.7 per million. Wound patients, especially chronic wound patients, are clearly a high-risk group for the acquisition, carriage, and dissemination of antibiotic resistant organisms. The cross contamination risks (nosocomial infections) include patient to patient exposure, handling of contaminated inanimate objects, transmission or carrier by health care personnel, long-term use of antibiotics (resulting in bacterial resistance), and prolonged residence time in hospitals or nursing homes, which increases the probability of infection. In fact, some studies has shown that infections resulting from In-Hospital delays for elective surgery increase by 6.68% after 1 day and 20.56% after 10 days.

### Wound Disinfection Treatments

Several strategies have been employed to combat the significant infectious complication rates associated with wounds. However, to-date, these strategies have been mainly limited to improving surgical asepsis, surgical technique, and regimens of administration of peri-operative systemic antibiotics and local antibiotic irrigation procedures. New approaches are constantly being developed in hospitals including vacuum-sealed dressings, transparent film dressings, irrigation with antimicrobial agents, use of the port and cap, use of new agents such as deuteroporphyrin, gamma interferon (IFN-γ), silver sulfadiazone water soluble gel, geomagnetic therapy, and natural remedies such as milliacynic oil and lysozyme. Unfortunately, only few of these innovations have made a major impact on infection and fatality rates. However, at least some of these effective approaches have also been shown to have cellular toxicity issues. Indeed, most new approaches involve delivery of antimicrobial compounds in some form of salve or in dressings, to which many wound pathogens are resistant. Also, these treatments lend themselves to continued production of antibiotic resistant bacteria that will negatively affect future therapies against resistive bacteria such as Methicillin-Resistant *Staphylococcus aureus* (MRSA), Vancomycin-resistant *enterococci* (VRE) and *Acinetobacter baumanni.* It is estimated that *A. baumannii* accounts for 6% of Gram-negative infections in intensive care facilities in the U.S. with mortality rates as high as 54% having been reported. Isolation of MDR *Acinetobacter* soared from 6.7% in 1993 to 29.9% by 2004, emphasizing the need for newer and better drugs. Out of 1,040 antibiotics tested only 20 (1.92%) exhibited significant antimicrobial activity and only five compounds exhibited activity against the more resistant BAA-1605 *A baumanni.* Today, it is believed that *MRSA* and C. *difficile* are the leading causes of nosocomial infection in most parts of the world. In 2003, *S. aureus* was the leading pathogen associated with skin and soft tissue infections. In the last 20 years, *MRSA* has moved from an almost exclusively hospital-acquired pathogen (*HA-MRSA*) to a community-acquired pathogen, *CA-MRSA.*

Wound healing and "good" care of wounds has been synonymous with topical prevention and management of microbial contamination. Today's primary therapy involves the use of either topical application of antiseptics or systemic and topical use of antibiotics. The general perspective is that topical application of antibiotics to wounds has no advantages over the use of other antiseptic methods and may increase the risk of wound-healing by producing a sovereign bacteria that is resistant within the wound. The use of silver-based dressings for therapy against infections are widely used in chronic wound and burn therapy. There are several of these commercially available such as Acticoatt™, Aquacels Ag®, Contreet® Foam, PolyMem® Silver, Urgotul® SSD. Unfortunately, these silver containing dressings do not kill spores or biofilms and require long exposure times that may result in cytotoxicity to patient's own cells. The cytotoxic effect would explain, in part, the clinical observation of delayed wound healing or inhibition of wound epithelialization after the use of certain topical silver dressings.

The current FDA regulations state that to be rated as a disinfectant/sterilant, the compound has to be capable of destroying all microorganisms, including all bacterial spores. If used in an application with shorter exposure time, the disinfectant must destroy all viruses, vegetative bacteria, fungi, mycobacteria and some, but not all, bacterial spores. In addition, the disinfectant must be able to meet these microcidal requirements within a complex protein matrix such as that in a wound environment. If a compound does not meet these criteria then it can be registered as an antiseptic if it can kill 3 logs of a specified bacteria species and labeled as such. As used herein, the term "kill" refers to reducing the amount or the level of microorganism. Typically, the term "kill" refers to reducing at least 3 logs, typically at least 4 logs, often at least 5 logs, and more often at least 6 logs of microorganism within 15 minutes, typically within 10 minutes, often within 5 minutes, and more often within 1 minute. As used herein, the term "x logs" refers to 10^{x}. For example, if a composition is said to kill 6 logs of microorganism, it means that the amount of microorganism present after treatment is 1/10⁶ or less of the original (i.e., pretreatment or relative to the control) amount of microorganism.

There are a myriad of composition available that claim to kill 99.9% of *MRSA* and other vegetative bacteria and some spores on surfaces and skin (e.g., hand sanitizers). However, contaminated surfaces can contain millions of bacteria, some of which can be contained within complex matrices such as blood drops, thus making them difficult to kill. Other types of bacteria, such as *Bacillus subtilis,* form biofilms on surfaces of endoscopes and other medical devices for insertion into the body, which significantly reduces the antibacterial activity of most disinfectants. These disinfectants are often called sanitizers and claim to kill 99.9% of the bacteria present. Typically, however, none of these sanitizers will kill all bacteria that are present, especially when bacteria are present in high populations, contained within a complex matrix, existing as a biofilm, or in vegetative or spore form.

There are currently several topical antiseptics on the market that are used to treat or reduce bacterial infections in wounds. These include Betadine, which is a mixture of various compounds including Iodine, Polyhexanide (Prontosan®), chlorhexidine, hydrogen peroxide, as well as others. Most antiseptics are not suitable for continuous treatment of open wounds because they impede wound healing due to their cytotoxic effects on keratinocytes and fibroblasts. In general, current topical antiseptics have limited bactericidal effect (e.g., only 3 log reduction of bacteria in 30 minute exposure) and nearly all have some cytotoxicity that varies with concentration and application time. Silver Nitrate solutions are in the antiseptic category and its cytotoxicity is well known.

There are primarily five high level disinfectants/sterilants in use today. These include glutaraldehyde, orthopthalaldehyde, hypochlorite, hydrogen peroxide, and peracetic acid. The aldehydes are generally highly toxic and take a very long time to affect a ≥99.9999% (or 6 log kill) of spores. The most successful high level disinfectants used today appears to be oxidizers such as Hypochlorites, Hydrogen Peroxide and Peracetic acid. It is believed that the reactive advantage for disinfection by oxidation is the non-specific free radical damage to all components of the microbe, including proteins, lipids, and DNA. Therefore, microbial resistance to oxidation at high enough solution concentration is virtually non-existent. Safe and non-toxic concentrations of hydrogen peroxide are not capable of killing high populations of microbes. Hypochlorous acid, which is formed by PMN by myeloperoxidase-mediated peroxidation of chloride ions, is easily neutralized at physiological pH by nitrite, a major end-product of cellular nitric oxide (NO) metabolism, thereby reducing hypochlorous acid's bactericidal effects. Due at least in part to this neutralization in situ, it has been shown that hypochlorous acid is not as effective as silver sulfadiazine, a common topical wound sanitizer.

### Microbial Infection in General

Systemic illness caused by microbial invasion of normally sterile or physical barrier parts of the body, such as the skin, is referred to as "sepsis." Any opening of the sterile or physical barrier body parts (i.e., a wound) must therefore be quickly and effectively repaired in order to restore tissue integrity and function. Quite often proper healing is impaired with devastating consequences such as sepsis that can lead to severe morbidity and possibly mortality. Some studies indicate an incidence of 3 cases of sepsis per 1000 population per year or about 750,000 cases of sepsis a year in the United States.

Very few pathogens, other than parasites such as malaria, multiply preferentially in the bloodstream. Sepsis thus generally originates from a breach of integrity of the host barrier systems, either physical (such as damage or compromise to the skin and intact anatomical systems) or immunological (failure of the immune system to effectively recognize and eradicate an infective microorganism), and direct penetration of the pathogen into the bloodstream, creating the septic state.

Currently, there are no rapid and reliable techniques for differentiating a microbial from a non-microbial cause of systemic inflammation, and there are no rapid techniques for readily identifying the causative organism(s). Regardless of availability of rapidly identifying the cause of systemic inflammation due to wound, a broad spectrum disinfectant and wound healing would allow wound healing without the need for a immediate and definitive identification of the infectious organisms.

Accordingly, there is a need for a composition having a broad spectrum disinfectant and/or would healing activity to reduce the incidence of sepsis resulting from a wound in a subject.

### Compositions of the Invention

Some aspects of the invention is based on a surprising and unexpected discovery by the present inventors that peroxy α-keto carboxylic acids (PKCAs) can be used to treat wound, promote wound healing, and have antimicrobial properties.

Representative examples of suitable PKCA for the invention are disclosed in a commonly owned U.S. Patent Application Nos. 12/618,605 filed November 13, 2009, and 12/760,940 filed April 15, 2010 as well as in a commonly owned U.S. Provisional Patent Application No. 61/444,111 filed February 17, 2011, all of which are incorporated herein by reference in their entirety.

In some embodiments, compositions of the invention comprise a mixture of an α-ketoester and PKCA. α-Ketoesters are ester compounds where the α-position (i.e., the 2-position or the position next to the ester functional group) of the molecule is a carbonyl group. In some instances, the α-ketoester is an alkyl α-ketoester. An alkyl α-ketoester refers to α-ketoester in which the ester functional group is an alkyl ester. In some cases within these instances, the alkyl α-ketoester is an ethyl α-ketoester or an alkyl pyruvate. In one particular instance, α-ketoester is ethyl pyruvate.

Surprisingly and unexpectedly, the present inventors have discovered that α-ketoesters have antimicrobial activity on their own. Furthermore, the presence of α-ketoesters in the mixture enhances tissue penetration of PKCA. In some instances, α-ketoesters also diminish cell's toxic anti-inflammatory response to pathogens. More surprisingly and unexpectedly, the present inventors have discovered that the combination of an α-ketoester and PKCA affords a synergistic antimicrobial activity as well as synergistic effect on wound treatment/healing and synergistic penetration of tissues.

It was discovered that compositions of the invention comprising a PKCA or a mixture of PKCA and α-ketoester simultaneously disinfect, stimulate immune cellular metabolism, decrease cellular hypoxia and promote early wound debridement while protecting against DNA damage. In some embodiments, compositions of the invention also include hydrogen peroxide and the carboxylate anion of the α-ketocarboxylic acid of the corresponding PKCA. These compounds are believed to exist in equilibrium with PKCA and thus are expected to be present and exert at least some activity within the mixture to disinfect and heal wounds according to each of their metabolic and cellular abilities. Without being bound to any theory, it is believed that the presence of an α-ketoester (such as the α-ketoester of the PKCA used) reduces inflammation of the cell that often results from the by-products of the dead bacteria.

The disinfecting capability of a pyruvate PKCA compound has been tested and shown to be a disinfectant/sterilant as defined by the Environmental Protection Agency (EPA). This test is the ASTM-E2197 method, which requires proof of killing 6 logs of *Clostridium difficile* spores on a stainless steel surface within a very high protein environment in 10 minutes or less. Without being bound by any theory, it is believed that the antimicrobial property of PKCA is due to the peracid functional group, and therefore PKCA is expected to eliminate or minimize any possibility of developing resistance by microorganisms. The other chemical compound that may be present within the compositions of the invention (e.g., α-ketoester, hydrogen peroxide, and/or carboxylic acid, etc.) may have wound healing properties, although they themselves can also have antimicrobial property.

In some embodiments, compositions of the invention include PKCA and optionally the corresponding α-ketoacid of the PKCA and/or the anion of such α-ketoacid. For example, if peroxy pyruvic acid (i.e., a compound of the formula HOOC(=O)C(=O)CH₃) is the PKCA in the composition, the resulting composition can optionally also include pyruvic acid and/or the anion of pyruvic acid. This particular PKCA composition is hereinafter referred to as perpyruvic acid or PPA. Similarly, the composition comprising peroxy α-ketobutyric acid as the PKCA is sometimes referred to herein as simply POKBA and the composition comprising peroxy α-ketovaleric acid is sometimes referred to herein as simply POKVA.

In other aspects of the invention, compositions of the invention consists of a PKCA, an α-ketoester, and optionally one or more of the following: the parent carboxylic acid of PKCA and/or a salt thereof, decarboxylated derivative of PKCA, and hydrogen peroxide. The term "parent carboxylic acid of PKCA" refers to a carboxylic acid having the same number and carbon atom connections as that of PKCA except that the peroxy (-OOH) moiety is replaced by a hydroxyl (-OH) moiety. The term "decarboxylated derivative of PKCA" or "decarboxylated PKCA" or other similar terms are used interchangeably herein and refers to a compound in which the terminal peroxy carboxylic acid moiety has been removed, e.g., by hydrolysis. For example, a decarboxylated derivative of peroxy pyruvic acid (HOOC(=O)C(=O)CH₃) refers to acetic acid (HOC(=O)CH₃).

### α-Ketoacid Anions

Studies have shown that cytotoxic oxidizers are released by cells in the inflammatory phase of a wound. These oxidizers are known as the reactive oxygen species (ROS) and include a singlet oxygen, superoxide anion, hydrogen peroxide, hydroxyl radical, and nitric oxide (NO). It is believed that one of the primary functions of the ROS is to kill microbial contamination. When a subject suffers a wound, polymorphonuclear leukocytes (PMN) gather at the wound site and release ROS. It was thought that the ROS species are only involved in killing bacteria within the wound. However, if a wound is exposed to these ROS for a prolonged period (e.g., because of inflammation due to infection), then there is a delay in wound healing due to ROS's toxicity to healthy cells.

Typically, the oxygen demand in wounds exceeds supply (hypoxia) for a few days following injury and α-keto pyruvate is well known for its protective properties against hypoxia. It has been shown that pyruvate improves the adaptive response and resistance to hypoxia in a multitude of metabolic ways. For example, pyruvate reduces oxidative stress (over production of oxidative molecules) caused by the release of lipopolysaccharide (LPS) from dead bacteria cell membranes (inflammation).

Pyruvate is believed to be one of the primary sources of energy for hypoxic cells. It is also believed that pyruvate reduces DNA damage during hypoxia conditions. Lactate, the end product of aerobic glycolysis and reduction of pyruvate, may play a role in cellular, regional, and whole body metabolism. Pyruvate in hypoxic cells then becomes an indirect metabolic contributor to other cellular functions through lactate signaling for collagen deposition and angiogenesis in wound healing. Furthermore, it has been shown that pyruvate and lactate together play a role in the up regulation of VEGF for an angiogenic response to hypoxia in wounds.

### Hydrogen Peroxide

Of all the cytotoxic oxidizers produced by PMN cells in a wound such as singlet oxygen, superoxide anion, hydroxyl radical, nitric oxide and H₂O₂, it has been shown that only H₂O₂ has a long enough half-life to accumulate in the culture medium of cells. It has also been shown that H₂O₂ becomes a metabolic initiator for the stimulation of compounds essential for the wound healing process under certain conditions. For example, it has been demonstrated that H₂O₂ stimulates human macrophages to release high levels of vascular endothelial growth factor (VEGF). It has also been shown that hydrogen peroxide stimulates re-epithelialization of wounds, wound coagulation of neutrophils, and monocyte adhesion to the extracellular matrix and endothelial cells. In addition, hydrogen peroxide plays a role as a messenger in stimulating growth factors required for wound healing such as platelet derived growth factor (PDGF), tissue growth factor (TGF), epidermal growth factor (EGF), and vascular endothelial growth factor (VEGF).

The external addition of high levels of H₂O₂ to diminish microbial infection is known to be toxic to cells and therefore not recommended for continual use. However, the cells themselves produce very small extracellular H₂O₂ concentration gradients. In some embodiments of the invention, compositions of the invention comprise a sufficient amount of H₂O₂ needed to kill 6 logs (i.e., 10⁶) of bacteria, e.g., in the micro molar concentration which is also a sufficient concentration to stimulate wound healing.

### α-Ketoesters

In some aspects, compositions of the invention comprise α-ketoesters. α-Ketoesters are ester compounds where the α-position (i.e., the 2-position or the position next to the ester functional group) of the molecule is a carbonyl group. In some embodiments, the α-ketoester is an alkyl α-ketoester. An alkyl α-ketoester refers to α-ketoester in which the ester functional group is an alkyl ester. In some instances within these embodiments, the alkyl α-ketoester is an ethyl α-ketoester. In one particular embodiment, α-ketoester is ethyl pyruvate. However, it should be appreciated that the scope of the invention is not limited to any particular α-ketoester. The present inventors have discovered that α-ketoesters sublimate the unpleasant odor of the PKCA. Without being bound by any theory, it is also believed that *α-Ketoesters* such as ethyl pyruvate stabilize the PKCA solution by stabilizing the hydrogen peroxide that is present within the solution.

The amount of α-ketoester present relative to the PKCA in compositions of the invention typically ranges from about 0.1 mol% to about 20 mol%, often from about 0.25 mol% to about 15 mol%, and more often from about 1 mol% to about 5 mol%. Alternatively, the amount of α-ketoester present in compositions of the invention ranges from about 1 % by weight to about 30 % by weight, typically from about 1.5 % by weight to about 15 % by weight, and often from about 5 % by weight to about 12 % by weight of PKCA.

### Utility

Conventionally widely used wound antiseptics do not always kill a sufficient amount of bacteria or spores required to promote wound healing and are often cytotoxic at longer term exposure. Some studies have been shown that irrigation of open fracture wounds with antibiotic solution offers no significant advantages over the use of a nonsterile soap solution and may actually increase cytotoxicity and inhibit wound healing. And the use of topical and systemic antibiotic treatment can sometimes lead to multi-drug resistant organisms.

Currently, there is no sufficiently suitable composition that is available for treating a wound with both an effective broad spectrum antimicrobial activity and effective enhanced healing property. There are treatments with wound healing stimulators subsequent to or in conjunction with cytotoxic antiseptics or antibiotics. While it may be possible to combine an antimicrobial compound such as antimicrobial nucleotides, polysaccharides, and/or proteins (generally referred to as growth factors), and an antioxidant for use of as molecular stimulators for wound healing, the cost of antimicrobial compound is relatively costly to produce and difficult to stabilize in the presence of an antioxidant.

Compositions of the invention are of a relatively low cost and stable broad spectrum antimicrobial compositions that are substantially not cytotoxic, and enhance wound healing. In addition, compositions of the invention are effective against biofilms such as those formed in chronic wounds. The present inventors have developed a family of PKCAs for use as a high level disinfectant/sterilant of vegetative bacteria, spores and biofilms and are described in the above incorporated by reference and commonly assigned patent applications. Table A below illustrates the ability of one particular PKCA compound to kill (i.e., reduce the amount or the level of) vegetative bacteria and spores at the concentration or amount acceptable to be called disinfectants and sterilants.

**Table A. Antimicrobial Activity of PPA**

| Microorganism | Log₁₀ kill | Time | Concentration |
|---|---|---|---|
| *Pseudonmas Aerginosa* | ≥ 6.0 | ≤ 1 min | 500 ppm |
| MRSA | ≥ 6.0 | ≤ 15 sec | 100 ppm |
| *Acinetobacter baumanii* | ≥ 6.0 | ≤ 15 sec | 100 ppm |
| *Candida albicans* | ≥ 6.0 | ≤ 15 sec | 100 ppm |
| *Clostridium difficile* spores | ≥ 6.0 | ≤ 5 min | 1,500 ppm |
| *Bacillus Subtillis* Biofilm | ≥ 5.0 | < 10 min | 4,000 ppm |
| Influenza Virus | ≥ 5.0 | ≤ 1 min | 300 ppm* |
| *Buckholder pseudomallei* | ≥ 6.0 | ≤ 1 min | 50 ppm |
| *Aspergillus* spores | ≥ 6.0 | < 10 min | 3,500 ppm |

| | | | |
|---|---|---|---|
| * lower concentration not tested | | | |

First three are antibiotic resistant bacteria.
*A. baumanii* is highly resistant to most antibiotics and prevalent in combat wounds.
*C*. *albicans* is fungus that causes oral and genital infections in humans.
*C*. *difficile* spores is very difficult to kill bacterial spore that causes a pathogenic infection which can be fatal.
*B. Satbtilis* is very difficult to kill bacteria spore that causes biofilms.
Influenza virus is commonly known as the flu virus.
*B. pseudomallei* is often considered as a potential bioterrorism organism that literally "spits" antibiotic out.
*Aspergillus* spore is fungal spore responsible for high mortality in burn wounds and is not significantly responsive to most conventional antiseptics.

It has been discovered by the present inventors that unlike most other peroxy carboxylic compounds, the PKCA compounds do not require an acid catalyst for efficient synthesis. Without the need for or the use of a toxic catalyst for synthesis, compositions of the invention have substantially no cytotoxic property when used in therapeutically effective amounts. In some embodiments, PKCA compound may be in equilibrium with the corresponding α-keto acid, hydrogen peroxide, and the corresponding decarboxylated carboxylic acid, some of which are beneficial to healing of the wound. Many of the parent compounds of the PKCA's (e.g., pyruvic acid) are present within nearly all living cells and play significant roles in essential cellular metabolism. For example, the parent compounds of peroxypyruvic acid (i.e., pyruvic acid), peroxy Oxaloacetate (i.e., oxalic acid), peroxy α-keto glutarate (i.e., α-keto glutaric acid), are key compounds within the TCA (i.e., Tricarboxylic cycle also known as the Krebs cycle), the predominant energy producing mechanism for cellular metabolism. The parent compound of peroxy α-keto butyric acid (i.e., α-keto butyric acid) is involved in the metabolic production of Succinyl-CoA, which is also used in the TCA cycle. α-Keto valeric acid, the parent compound of peroxy α-keto valeric acid, is one of the key intermediates in protein synthesis and the biosynthesis of the amino acids such as leucine and valine. α-Keto valeric acid is also involved in gluconeogenesis in cells. Pyruvate is involved in producing energy for hypoxic cells during wound healing through glycolysis. The potential harmful effects of the ROS can be mediated by the α-keto acid. In addition, pyruvate also has protective effect on DNA damage during hypoxia and is an indirect metabolic contributor to collagen deposition and angiogenesis in wound healing. Furthermore, pyruvic acid accelerates the debridement of the dead skin in both wounds and burns.

Topical antiseptics should have toxicity to bacteria but not to underlying tissues, and ideally, they should also preserve or enhance host defense against infection. Some aspects of the invention provide methods for treating a wound, e.g., surgical, traumatic, chronic and burn wounds). In some embodiments, methods of the invention include healing and rapidly killing (i.e., reducing the level and/or the amount of or eliminating completely of) microorganisms such as, but not limited to, viruses, vegetative bacteria, fungi, bacteria (e.g., mycobacteria) and spores. Unlike other conventional antiseptics available today, in some embodiments compositions of the invention eliminate substantially all microorganisms and enhance the wound healing process. It should be appreciated that each wound type may be unique in the optimum requirements for the PKCA and/or the α-ketoester used in treating wound. Some of the therapeutic uses of compositions of the invention include, but are not limited to, (i) use as an irrigation solution during early treatment of traumatic or acute wounds; (ii) use as an irrigation solution following completion of a surgical procedure; (iii) preventing nosocomial infections after surgery and treatment of acute wounds; (iv) treating wounds where biofilm colonization and/or antibiotic resistant infections have or are expected to resulted in a slow healing or chronic wound; (v) use in debridement, antimicrobial therapy and/or healing of burns, including chemical burns; (vi) treating infected decubitus ulcers; (vii) treating foot ulcers; (viii) treating venous ulcers; and (ix) treating any type of wound resulting from laser treatments, e.g., for the removal of scar and wrinkles. In general, any kind of skin or tissue damage can be treated with a composition of the invention including, but not limited to, sunburn, abrasions, surgical wounds, puncture wounds, etc.

A therapeutically effective amount of a composition of the invention is generally the amount that is sufficient to prevent and/or reduce further injury to wounds and/or increase the healing rate of the wounds. A therapeutic agent for wound treatment optionally can also include other wound treatment compounds such as the metabolic growth factors, antibiotics and/or antimycotics and stimulators. Compositions of the invention can be administered using a carrier solution. Exemplary suitable carrier solutions include, but are not limited to, physiological pH buffers, isotonic liquids and media. Compositions of the invention can also be formulated as a cream, gel, ointment, lotion, patch, and the like. Ointments and creams can, for example, be formulated with an aqueous or oily base with the addition of suitable thickening and/or gelling agents. Lotions can be formulated with an aqueous or oily base and will in general also contain one or more emulsifying agents, stabilizing agents, dispersing agents, suspending agents, thickening agents, or coloring agents.

Compositions of the invention can also be formulated for aerosol administration, particularly as a spray on administration. The composition will generally have a small particle size for example of the order of five (5) microns or less. Such a particle size can be obtained by means known in the art, for example by micronization. The composition can be provided in a pressurized pack with a suitable propellant such as a chlorofluorocarbon (CFC), for example, dichlorodifluoromethane, trichlorofluoromethane, or dichlorotetrafluoroethane, or carbon dioxide or other suitable gas. The aerosol can conveniently also contain a surfactant such as lecithin. The dose of composition can be controlled by a metered valve or simply by the amount of spray time.

The amount of composition used in wound treatment can vary depending on a wide variety of factors including, but not limited to, the type and condition of the wound being treated, the size of the wound, age of the subject, amount of contamination present in the wound, weight of the subject, the form of administration and the particular PKCA and α-ketoester (if present) chosen, etc. In general, the physician can readily determine the amount of the composition of the invention that will be most suitable for a particular wound treatment for the patient.

As an example, a higher concentration of the composition of the invention may be more appropriate for treating a chronic wound than traumatic wound. Therefore, the amount of composition used would vary depending on the wound requirement. In some embodiments, the amount of PKCA in compositions of the invention ranges from 0.01 mM to about 1 M, typically from about 1 mM to about 0.5 M, often from about 10 mM to about 250 mM. Generally, for all types of wounds, the amount of PKCA in compositions of the invention used is from about 0.1 mM to about 200 mM. In one particular embodiment for treating all types of wounds, the amount of PKCA in the composition of the invention ranges from about 0.96 mM to about 192 mM.

For chronic wound treatment, the concentration of the PKCA in the composition of the invention is typically from about 0.1 mM to 1 M, often from about 1 mM to about 0.5 M, and more often from about 10 mM to about 250 mM. In one particular embodiment for chronic wound treatment, the concentration of PKCA in the composition of the invention ranges from about 115 mM to about 154 mM. In another embodiment, the concentration of PKCA in the composition of the invention ranges from about 82 mM to about 96 mM. Still in another embodiments, the concentration of PKCA in the composition of the invention ranges from about 38 mM to about 76 mM.

For non-chronic wound treatment, the concentration of the PKCA in the composition of the invention ranges typically from about 0.01 mM to about 1 M, often from about 0.1 mM to about 500 mM, and more often from about 0.1 mM to about 250 mM. In one particular embodiment for non-chronic wound treatment, the concentration of PKCA in the composition of the invention ranges from about 38 mM to about 77 mM. In another embodiment, the concentration of PKCA in the composition of the invention ranges from about 19 mM to about 38 mM. Still in another embodiments, the concentration of PKCA in the composition of the invention ranges from about 4.2 mM to about 8.5 mM. Yet in other embodiments, the concentration of PKCA in the composition of the invention rangesfrom about 0.96 mM to about 2.1 mM.

As stated above, in some embodiments, the parent α-keto acid and/or the anion thereof of the PKCA may be present in compositions of the invention. When and if present, the amount of the parent α-keto acid and/or the anion thereof of the PKCA is typically in an equilibrium concentration amount. Alternatively, when and if present, the parent α-keto acid and/or the anion thereof of the PKCA present in compositions of the invention ranges from about 0.01 mM to about 10 M. In one particular embodiment, the amount of parent α-keto acid and/or the anion thereof of the PKCA present in compositions of the invention ranges from about 12.4 mM to about 7,352 mM. In another embodiment, the amount of parent α-keto acid and/or the anion thereof of the PKCA present in compositions of the invention ranges from about 2.5 mM to about 6.2 mM. Still in another embodiment, the amount of parent α-keto acid and/or the anion thereof of the PKCA present in compositions of the invention ranges from about 0.62 mM to about 1.2 mM. Yet in another embodiment, the amount of parent α-keto acid and/or the anion thereof of the PKCA present in compositions of the invention ranges from about 0.062 mM to about 0.31 mM.

As stated above, in some embodiment, hydrogen peroxide may also be present in compositions of the invention. When and if present, the amount of hydrogen peroxide is typically in an equilibrium concentration amount. Alternatively, when and if present, the amount of hydrogen peroxide in compositions of the invention ranges from about 0.01 mM to about 10 M, typically from about 0.1 mM to about 5 M, and often from about 1 mM to about 5 M. In one particular embodiment, the amount of hydrogen peroxide present in compositions of the invention ranges from 4.9 mM to about 2940 mM. In another embodiment, the amount of hydrogen peroxide present in compositions of the invention ranges from about 586.4 mM to about 785.3 mM. Still in another embodiment, the amount of hydrogen peroxide present in compositions of the invention ranges from about 418.2 mM to about 489.6 mM. Yet in another embodiment, the amount of hydrogen peroxide present in compositions of the invention ranges from about 193.8 mM to about 387.6 mM.

For treating non-chronic wound, in one particular embodiment, the amount of hydrogen peroxide present in compositions of the invention ranges from about 193.8 mM to about 392.7 mM. In another embodiment, the amount of hydrogen peroxide present in compositions of the invention ranges from about 43.3 mM to about 96.9 mM. Still in another embodiment, the amount of hydrogen peroxide present in compositions of the invention ranges from about 4.9 mM to about 21.4 mM.

Some aspects of the invention provide compositions that comprise in addition to PKCA an α-ketoester. In such compositions, the amount of α-ketoester ranges from about 0.01 mM to about 1 M, typically from about 0.1 mM to about 0.5 M, often from about 0.5 mM to about 250 mM. In one particular embodiment, the amount of α-ketoester ranges from about 0.72 mM to about 172 mM.

For chronic wound treatment, the amount of α-ketoester in compositions of the invention ranges from about 0.1 mM to about 500 mM, typically from about 1 mM to about 250 mM, and often from about 10 mM to about 100 mM. In one particular embodiment, the amount of α-ketoester in compositions of the invention ranges from about 34 mM to about 46 mM. Yet in another embodiment, the amount of α-ketoester in compositions of the invention ranges from about 23 mM to about 28.6 mM. Still in another embodiment, the amount of α-ketoester in compositions of the invention ranges from about 11.5 mM to about 17.2 mM.

For non-chronic wound treatment, the amount of α-ketoester in compositions of the invention ranges from about 0.01 mM to about 500 mM, typically from about 0.05 mM to about 250 mM, often from about 0.1 mM to about 100 mM. In one particular embodiment, the amount of α-ketoester in compositions of the invention ranges from about 10 mM to about 11.5 mM. In another embodiment, the amount of α-ketoester in compositions of the invention ranges from about 7.2 mM to about 8.6 mM. Yet in another embodiment, the amount of α-ketoester in compositions of the invention ranges from about 4.3 mM to about 6.4 mM. Still in another embodiment, the amount of α-ketoester in compositions of the invention ranges from about 0.29 mM to about 2.6 mM.

In some embodiments, compositions of the invention can kill at least 10⁵ amount of microorganisms within 10 minutes at a concentration of about 5,000 ppm or less often at least 10⁶ microorganisms within 10 minutes at a concentration of about 5,000 ppm including microorganism spores and microorganisms in biofilms. As used herein, the term "microorganism" includes bacteria, virus, fungi, algae, prion, and other pathogenic organisms known to one skilled in the art. Typically, the term microorganism refers to bacteria, virus, and fungi.

In other embodiments, compositions of the invention have microorganism kill activity of at least log 5 within 10 minutes, typically within 5 minutes and often within 1 minute at a concentration of 4,000 ppm. Still in other embodiments, compositions of the invention have microorganism kill activity of at least log 6 within 10 minutes, typically within 5 minutes and often within 1 minute at a concentration of 4,000 ppm. Yet in other embodiments, compositions of the invention have microorganism kill activity of at least log 6 within 10 minutes at a concentration of about 4,000 ppm, typically at 3,000 ppm, often at 1,000 ppm, and more often at 500 ppm.

Surprisingly and unexpectedly, it has been found by the present inventors that compositions of the invention can also kill microorganism spores and biofilms including, but not limited to, those disclosed herein. See, for example, Table A above. Conventional antiseptics/detergents typically cannot kill microorganism spores and/or biofilms in an effective manner. In contrast, compositions of the invention have a broad spectrum activity and can effectively kill at least 70%, typically at least 80%, often at least 90%, more often at least 95%, and still more often substantially all of bacteria in biofilm within 10 minutes at a concentration of about 5,000 ppm.

Additional objects, advantages, and novel features of this invention will become apparent to those skilled in the art upon examination of the following examples thereof, which are not intended to be limiting. In the Examples, procedures that are constructively reduced to practice are described in the present tense, and procedures that have been carried out in the laboratory are set forth in the past tense.

### EXAMPLES

### EXAMPLE 1

### Disinfection of Spores on Medical Devices

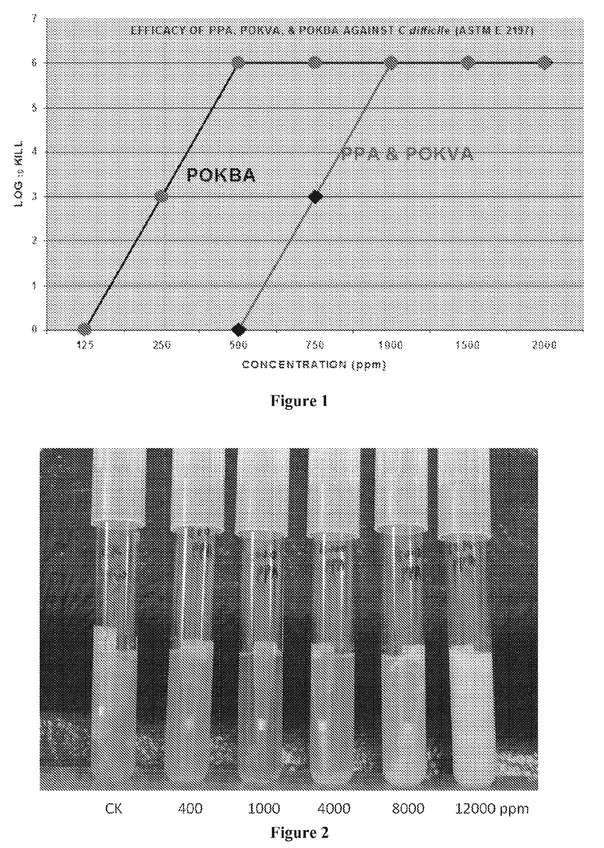

This example demonstrates that the sporicidal efficacy of the PKCA compounds in a dry, high protein environment, using the method described in the ASTM E-2197 procedure. Figure 1 illustrates sporicidal activity of peroxy α-keto pyruvic acid (PPA), peroxy α-keto valeric acid (POKVA), and peroxy α-keto butyric acid (POKBA). Each of these solutions was challenged to kill 6 logs of C. *difficile* spores in 10 minutes in a high protein environment. The concentrations required were 1000 ppm (8.5 mM) for PPA and POKVA and 500 ppm (4.2 mM) for POKBA. In addition, 3 logs of C. *difficile* spores were killed with a PPA and POKVA at a concentration of 750 ppm (6.3 mM) and with POKBA at 250 ppm (2.1 mM). These concentrations of PKCA are equivalent to α-keto acid concentrations of 12.4 mM (1000 ppm), 9.3 mM (750 ppm), and 3.1 mM (250 ppm).

### EXAMPLE 2

### Disinfection of Biofilms on Medical Devices

The efficacy of PKCA against surface biofilm formation was tested by the AOAC 966.04 procedure. This procedure tests a candidate disinfectant against *Bacillus subtillus* spores dried onto ceramic penicylinders where they can form a biofilm. Briefly, a dilution of spore suspension in sterile distilled water is prepared at a final concentration equal to 1-4x10⁷ CFU/mL. Using a sterilized hook, sterile penicylinders are placed in the prepared dilution and mixed well and then allowed to incubate for 10-15 minutes. Afterwards, the cylinders are removed, placed onto a sterilized screen in a sterile petri dish and then placed in a desiccator for at least 12-24 hours or until time of use. For disinfectant testing, the contaminated penicylinders and a non-contaminated control cylinder are placed into vials containing the PKCA mixture and allowed to set for 10 minutes. Afterwards, the number of spores are enumerated by placing a single cylinder into 10 mL of anaerobic Brucella broth, sonicated, and the appropriate dilution made on agar plates based upon the expected count (typically spiral plate 50 µL of a 1:1000 dilution). The cylinders should contain 10⁶ cfu/cylinder and subsequent loss in count from exposure to the PKCA mixture reflects the log kill of the spores. The results showed that each of the three PKCA solutions containing PPA, POKBA, and POKVA concentrations of 169 mM (2000 ppm) were able to kill ≥ 5.0 logs of *Bacillus subtilis* on dried ceramic cylinders in 15 minutes.

### EXAMPLE 3

### Materials and Methods

### Strains and growth condition

*Pseudomonas aeruginosa* PAO1 (ATCC number: BAA-47), *Enterococcus faecalis* V583 (ATCC number: 700802), and *Staphylococcus aureus* (ATCC number: 700699) were grown in Tryptic soy broth (TSB, Sigma Chemical Co., St. Louis, MO, USA) medium at 37 °C with shaking for 16 hr.

### Chemical treatment on biofilm formation

Bolton broth (Oxoid Ltd, Basingstock, Hampshire, England) and Bovine plasma (Biomeda, Foster City, CA, USA) were used for multi-species biofilm formation. *Pseudomonas aeruginosa* PAO1, *Enterococcus faecalis* V583, and *Staphylococcus aureus* grown on TSB agar plates were inoculated into TSB broth and grown at 37 °C in a shaker for 16 hr. An aliquot was diluted in TSB broth to a series of dilutions for each individual bacteria type. The diluted bacteria were plated out to count colony forming units (CFU). They were further diluted to1 x 10⁶ cfu/mL. and mixed equally as inoculums. Bolton broth with 50% plasma was used for biofilm formation media. Glass 16 x 150 mm test tubes with caps were autoclaved, and 7 ml biofilm formation media aseptically dispensed in each tube. The normalized cultures of the three bacteria were mixed and 10 µl of the combined and normalized culture 1 x 10⁶ CFU/mL were inoculated into glass tubes. This was done by ejecting the pipette tips into the tubes. The pipette tip acts as a surface for biofilm formation. To the tubes, PPA/PPA-EP were added at 0 ppm, 400 ppm and 4000 ppm, and grown at 37 °C in a shaker for 24 hr at 150 rpm. Biofilm formation was subjectively observed and the biofilms were collected. The set of tubes with biofilm were placed in an oven at 80 °C for 48 h to obtain a dry weight. The biomass dry-weight was measured as the difference of the total weight minus the empty tube weight measured before use. Tests were performed in triplicate for each treatment group. A separate set of tubes in triplicate was used for DNA extraction and quantitative PCR analyses as described below.

### PPA and PPA-EP incubation with formed biofilm

The formed biofilm were washed three times, and then treated with 8000 ppm and 16000 ppm PPA and PPA with ethyl pyruvate (EP), incubated at 37 °C with shaking (150 rpm) for 1 hr. The effects of PPA and PPA-EP incubation on formed biofilm were also evaluated using bacteria plate count. The biofilm were washed, sonicated for 10 min, and vortexed. The process was repeated one more time. The supernatants were then serially diluted for bacteria plate count.

### Designing specific primers for the three bacteria

Genome sequences of *Pseudomonas aeruginosa* PAO1 (GenBank number: AE004091), *Enterococcus faecalis* V583 (GenBank number: AE016830), and *Staphylococcus aureus* (GenBank number: BA000017) were downloaded from NCBI website. The individual genome sequence was used to BLAST against the whole publicized microbial genome sequences by using a Wnd-BLAST. The no-hit genes were used to design specific primers.

### Real-time PCR analysis

Biofilm samples were homogenized by using a Qiagen TissueLyser (QIAGEN, Santa Clara, CA, USA). A sterile 5 mm steel bead and 500 µL 0.1 mm glass beads were added to the tube with 500 µL TE buffer, and run at 30Hz for 5 min. Bacteria DNA from the biofilm samples were then extracted by using a QIAamp DNA mini kit (QIAGEN). DNA samples were quantified using a Nanodrop spectrophotometer (Nyxor Biotech, Paris, France), and were diluted to 20 ng/µl. DNA from three individual bacteria was also diluted to 20 ng/ul as positive control. Quantitative real-time PCR was used to assay specific gene levels for each bacterium representing the ratio of the three bacteria in biofilm samples. The levels of the genes were detected by using the Roche LightCycler 480 (Roche, Mannheim, Germany). LightCycler 480 SYBR Green I Master Kit (Roche) was used for 20 µl real-time PCR reactions. Each sample was assayed three times. The relative gene level of each sample was calculated and analyzed. In brief, the threshold cycle (Ct value) of the target genes in different samples was obtained after quantitative real-time PCR reaction. The normalizer DNA Ct value was subtracted from the gene of interest Ct (target gene) to produce the dCt value of the sample. The dCt value of the calibrator (the sample with the highest dCt value) was subtracted from every other sample to produce the ddCt value. Two to the -ddCt power (2-^{ddCt}) was taken for every sample as the relative gene levels. The gene expression level of each bacterium represents relative ratios of each bacterium within a given DNA extracted sample.

### Results

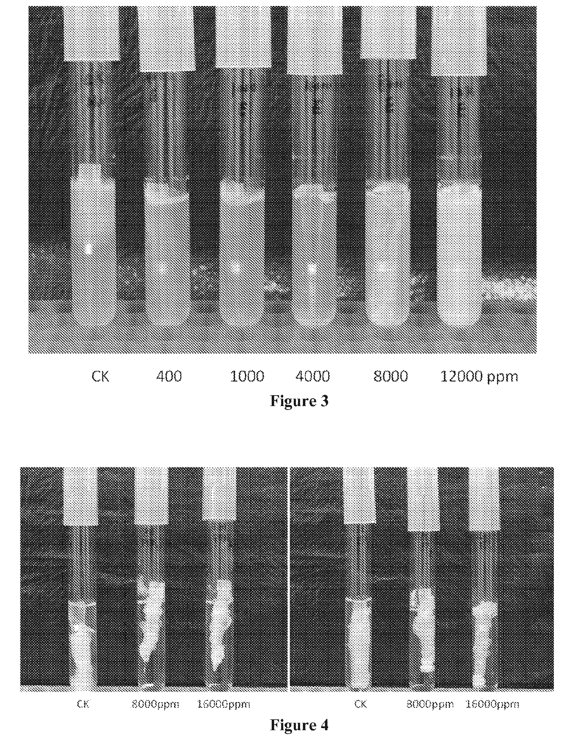

PPA and PPA-EP concentration of 400 ppm, 1000 ppm, 4000 ppm, 8000 ppm, and 12000 ppm were initially used for testing the correct concentration for further multi-chemical treatment on biofilm formation (Figures 2 and 3). PPA and PPA-EP demonstrated an obvious and significant inhibitory effect on biofilm formation. So 400 ppm and 4000 ppm were used as the final concentration for the assays. Subjective observations of the biofilm formation were made and the biofilm biomass dry-weight was also measured to provide a more objective measurement. All of the treatments, exhibited lower biomass formation, based upon dry-weight, than the control biofilms, and the decrease of the biomass correlated with the visible reduction of the biofilm formation (Table 1). Adding 400 ppm of PPA and PPA-EP, the biofilm biomass dry-weight was decreased by 42.2% and 52.8%. Adding 4000 ppm of PPA and PPA-EP, no biofilm growth was observed. The bacteria plate count further confirmed that 4000 ppm PPA and PPA-EP totally inhibit bacteria growth.

To further characterize the effects the chemicals on the individual bacterial populations within the multi-species biofilm, real-time PCR assay was developed. This test was used to compare untreated controls to the PPA and PPA-EP treated matrix, The results showed that PPA and PPA-EP did not completely inhibit growth of the 3 bacteria at 400 ppm but completely inhibited growth of the 3 bacteria at 4,000 ppm. See Table 1.

**Table 1. Data summary of chemical treatment on biofilm prevention (N/A = no counts)**

| Sample/Treatment | bacteria count (cfu/ml) | biofilm dry weight (mug ± SD) | *P. aeruginosa* | *E. faecalis* | *S. aureus* |
|---|---|---|---|---|---|
| CK (PPA) | 1.0 E11 | 112 ± 16.1 | 58.7% ± 7.2% | 16.9% ± 2.9% | 24.5% ± 0.7% |
| 400 ppm PPA | 4.0 E9 | 64.7 ± 2.5 | 62.3% ± 4.0% | 19.7% ± 1.8% | 20.5% ± 0.7% |
| 4000 ppm PPA | no growth | No Biofilm | NA | NA | NA |
| CK (PPA+EP) | 1.0 E11 | 115 ± 12.8 | 55.1% ± 0.01% | 12.3% ± 0.5% | 32.1% ± 4.2% |
| 400 ppm PPA+EP | 3.0 E8 | 54.3 ± 9.0 | 64.5% ± 5.7% | 15.2% ± 4.1% | 22.9% ± 1.6% |
| 4000 ppm PPA+EP | no growth | No Biofilm | NA | NA | NA |

In order to evaluate the effect of PPA/PPA-EP at eliminating formed biofilm, the 24 hr formed biofilm were treated with 8000 ppm and 16000ppm of PPA/PPA-EP for 60 min. By subjectively observation, PPA/PPA-EP does show the effect to eliminate formed biofilm (Figure 4). All of the treatments, exhibited more biofilm degradation, based upon dry-weight, than the control biofilms, and the decrease of the biomass correlated with the visible reduction of the biofilm (Table 2). Adding 8000 ppm of PPA and PPA-EP, the biofilm biomass dry-weight was decreased by 62.4% and 60.8%. Adding 16000 ppm of PPA and PPA-EP, the biofilm biomass dry-weight was decreased by 49.6% and 64.2%. The bacteria plate count further confirmed that 8000 ppm, 16000 ppm PPA and PPA-EP totally eliminate bacteria growth. To further characterize the effects the chemicals on the individual bacterial populations within the multi-species biofilm, real-time PCR assay was developed. This test was used to compare untreated controls to the PPA and PPA-EP treated matrix, The results showed that PPA and PPA-EP did not selectively eliminate these three bacteria (Table 2).

**Table 2. Data summary of PPA and PPA-EP treatment with formed biofilm**

| **Sample/Treatment** | **bacteria count (cfu/ml)** | **biofilm dry weight (mg ± SD)** | ***P. aeruginosa*** | ***E. faecalis*** | ***S. aureus*** |
|---|---|---|---|---|---|
| CK (PPA) | 1.0 E9 | 13.3 ± 2.1 | 13.1% ± 1.4% | 39.3% ± 0.4% | 47.6% ± 1.2% |
| 8000 ppm PPA | no growth | 5.0 ± 2.0 | 13.2% ± 1.4% | 29.0% ± 1.1% | 57.8% ± 5.7% |
| 16000 ppm PPA | no growth | 6.7 ± 2.3 | 17.8% ± 3.4% | 30.4% ± 8.4% | 51.8% ± 5.8% |
| CK (PPA+EP) | 1.0 E9 | 12.0 ± 1.7 | 19.5% ± 1.1% | 29.4% ± 0.1% | 51.2% ± 4.3% |
| 8000 ppm PPA+EP | no growth | 4.7 ± 2.1 | 20.5% ± 3.2% | 22.7% ± 0.3% | 56.7% ± 2.5% |
| 16000 ppm PPA+EP | no growth | 4.3 ± 2.1 | 17.8% ± 1.3% | 45.2% ± 0.9% | 36.9% ± 3.4% |

### Conclusion

PPA and PPA-EP have a broad range for inhibiting bacteria growth. At 8,000 ppm, PPA and PPA-EP eliminate formed biofilm within 60 min. At lower concentrations such as 400 ppm to 4,000 ppm, PPA and PPA-EP have a suppression effect on biofilm formation.

### EXAMPLE 4

This example demonstrates that the PKCA solutions kill bacteria in a simulated wound solution environment. The PKCA solution containing PPA was brought to the approximate physiological pH of 6.0 in a 50 mM phosphate buffer and tested for its ability to kill Methicillin-Resistant *Staphylococcus aureus* (MRSA) in the presence of 10% Fetal Bovine Serum (FBS). It was shown that 100 ppm (0.85 mM) of the PPA containing PKCA solution killed 6 logs of MRSA in one minute within the FBS solution.

All other PKCA efficacy studies against vegetative bacteria were done by a standard immersion test. This procedure involves producing a suspension of the test organism in sterile diluent comparable to a 0.5 McFarland standard (1-2 x 10⁸ CFU/mL). An aliquot of the suspension was pipetted into the PKCA mixture to be evaluated at a ratio of 1:100 (e.g., 30 µL suspension into 3mL of disinfectant mixture) and thoroughly mixed. An aliquot was removed from the PKCA mixture at desired exposure intervals and diluted to a ratio of 1:10 (e.g., 0.4 mL into 3.6 mL) in neutralizing broth and then spiral plated for counts. This procedure provides theoretical quantitation of a 6 log decrease in CFU/mL.

### EXAMPLE 5

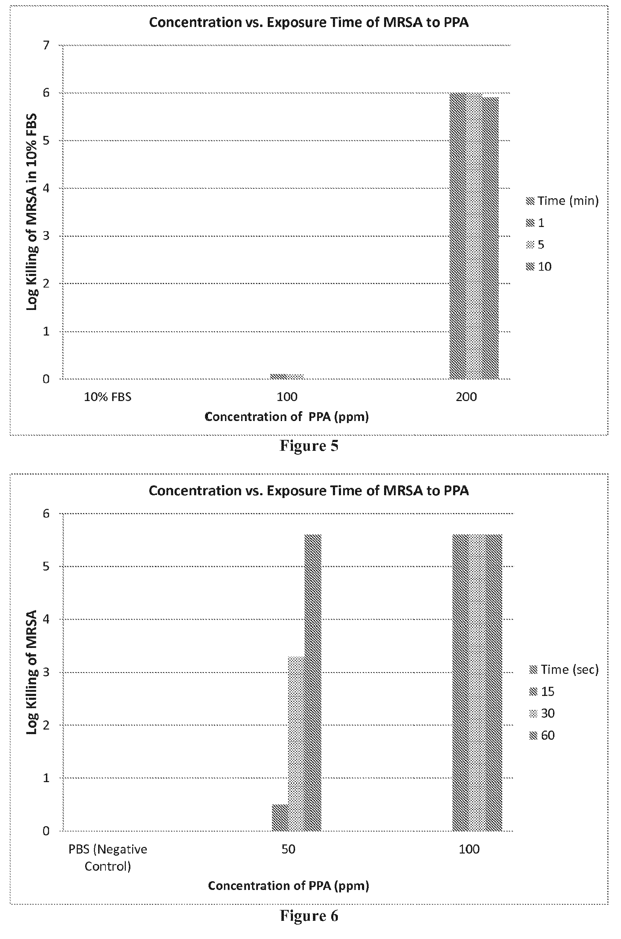

To determine the performance of the PKCA mixtures in a protein environment, the PPA mixture was tested for microcidal efficacy against MRSA for performance in a high protein environment and for a simple simulation of a wound environment. The PPA mixture was tested by the immersion test, as described above, against MRSA suspended in 10% Fetal Bovine Serum (FBS). The PPA mixture killed 6 logs of MRSA when exposed to 200 ppm of PPA in 10% FBS within 15 seconds (Figure 5). This was double the concentration of PPA required to kill MRSA suspended in water in 15 seconds. Therefore, an increase in concentration was required in a high protein environment, but the activity in the high protein environment for killing high populations of a MDRO was still fast and effective.

### EXAMPLE 6

The PPA mixture was also tested against MRSA suspended in a phosphate buffer to determine if buffered solutions of the PKCA mixtures at different pH's could be used for different phases of the wound healing process. The results of that testing demonstrated that PPA will kill 6 logs of MRSA in pH 6.0 phosphate buffer in 60 seconds at 50 ppm and in 15 seconds at 100 ppm (Figure 6).

### EXAMPLE 7

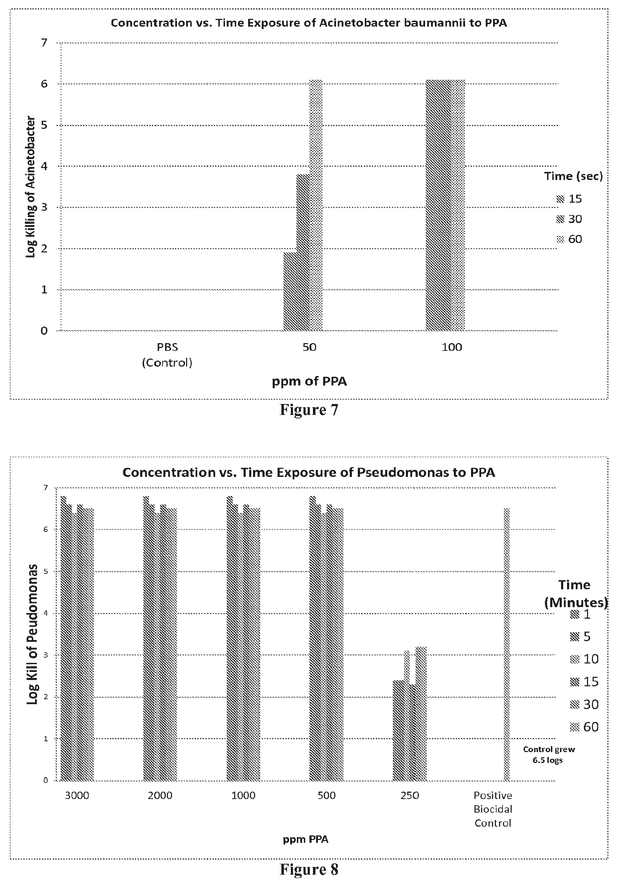

The PPA mixture was also tested against *Acinetobacter baumanii* suspended in a phosphate buffer to determine if buffered solutions of the PKCA mixtures at different pH's could be used for different phases of the wound healing process. The results of that testing demonstrated that PPA will kill 6 logs of *Acinetobacter baumanii* in 60 seconds with 50 ppm of PPA to kill 6 logs and only 15 seconds at 100 ppm (Figure 7).

### EXAMPLE 8

Experiments were performed on the biocidal efficacy of PPA against *Pseudomonas aeroginosa* suspended in 20% egg yolk in a citrate buffer, pH 6.8. The results demonstrated that PPA will kill *Pseudomonas aeroginosa* in a buffered high protein environment in 1 minute at 500 ppm but took 60 minutes to kill 6 logs at half that concentration (Figure 8).

Current real challenges in infectious wound healing include Candida and *Aspergillus* fungi and molds. *Aspergillus* spores in burn wounds have led to a 75% mortality rate in older patients with deep burn wounds. PPA has been shown to kill these spores in solution. Table 2 above shows that PPA will kill these fungi in less than 10 minutes and therefore indicates the ability to disinfect these infections in burn wounds before they become systemic.

These studies demonstrated the biocidal efficacy of PKCA mixtures in high protein and buffered solutions at different pH values. In addition, they demonstrate the prevention and destruction of simulated chronic wounds in-vitro. The contract research facility stated that after testing hundreds of compounds, that other than hypochlorite, the PKCA solution was the only compound they have seen that had a totally broad spectrum kill of the bacteria and prevented and dissolved the Biofilm. All of these examples and theoretical understanding of the chemistry demonstrate that PKCA compounds can be formulated according to an optimum pH to enhance wound healing and still disinfect the wound. It has been proposed recently that the tailoring of pH for the addition of treatment compounds would be an effective way to decrease wound healing time.

In summary, these examples demonstrate that the PKCA solutions can kill high levels of bacteria and spores in biofilms and in high protein environments. In many instances, the PKCA solutions also include the parent α-ketocarboxylic acids. The α-ketoester provides tissue penetration and anti-inflammatory capabilities to the wound treatment solution. Therefore, compositions of the invention are the only existing simple organic chemistry that would both disinfect a wound and enhance healing simultaneously.

### EXAMPLE 9

This example shows that the biocidal efficacy of the α-keto peracid (i.e., peroxy α-ketocarboxylic acid) compounds in a dry, high protein environment. The experiment was conducted following the method described in the ASTM E-2197 procedure. Figure 1 shows the results of three α-keto peracid solutions each of which contained either peroxy α-keto pyruvic acid (PPA), peroxy α-keto valeric acid (POKVA), or peroxy α-keto butyric acid (POKBA). These solutions were challenged to kill 6 logs of *C difficile* spores in 10 minutes within a high protein environment. The concentrations required were 1000 ppm (8.5 mM) for PPA and POKVA and 500 ppm (4.2 mM) for POKBA. In addition, 3 logs of C *difficile* spores were killed with a PPA and POKVA concentration of 750 ppm (6.3 mM) and with POKBA at 250 ppm (2.1 mM). These concentrations of α-keto peracids equate to α-keto acid concentrations of 12.4 mM (1000 ppm), 9.3 mM (750 ppm), and 3.1 mM (250 ppm).

### EXAMPLE 10

This example shows that the α-keto peracid solutions can kill biofilms. The biocidal efficacy testing of α-keto peracid compounds against biofilms was determined using the method described in the AOAC 966.04 procedure. The results showed that each of the three α-keto peracid solution containing PPA, POKBA, and POKVA at a concentration of 169 mM (2000 ppm) were able to kill ≥ 5.0 logs of *Bacillus subtilis* on dried ceramic cylinders in 15 minutes.

### EXAMPLE 11

This example shows that the α-keto peracid solutions can kill bacteria in a simulated wound solution environment. The α-keto peracid solution containing PPA was brought to the approximate physiological pH of 6.0 in a 50 mM phosphate buffer and tested for its ability to kill Methicillin-Resistant *Staphylococcus aureus* (MRSA) in the presence of 10% Fetal Bovine Serum (FBS). It was shown that 100 ppm (0.85 mM) of the PPA containing α-keto peracid solution killed 6 logs of MRSA in one minute within the FBS solution.

These examples demonstrate that the α-keto peracid solutions can kill high levels of bacteria and spores in biofilms and in high protein environments. In some instances, compositions of the invention comprise both the α-keto peracid and the parent alpha keto carboxylic acid. The alpha keto carboxylic acids are natural compounds found within nearly all living cells and have been implicated in potentially improved wound healing. By providing both the α-keto acids, and the peroxy form of these alpha keto acids, some compositions of the invention provide synergistic benefits to the cells.

### EXAMPLE 12

The PKCA compounds that are disclosed in the above disclosed commonly assigned U.S. Patent Applications and Provisional Patent Application. These PKCA compounds have been developed *inter alia* for use as a high level disinfectant/sterilant of vegetative bacteria, spores and biofilms. The present inventors have shown that PKCA compounds are effective in killing vegetative bacteria and spores at the level acceptable to be called sterilants/disinfectants.

In this study, ethyl pyruvate (EP) was added to a PKCA solution to determine if biocide efficacy was affected by the addition of EP. The EP was added at a 2% concentration to a solution comprising a PKCA compound. The amount of EP used was effective in substantially eliminating PKCA odor. As a control, 2% EP in water was tested for anti-microbial properties in the same manner as the PKCA-EP mixture. Surprisingly and unexpectedly it was discovered that the 2% EP control also killed bacteria. Examples of these tests are shown below.

MRSA was prepared as a suspension in sterile diluent that was comparable to a 0.5 McFarland standard (1-2 x 10⁶ CFU/mL). An aliquot of the MRSA suspension was added to the PKCA-EP mixture and the EP control solution at a ratio of 1:100 (e.g., 30 µL suspension into 3 mL of the test solutions) and thoroughly mixed by vortex. After 10 minutes, an aliquot of each test sample was diluted at a ratio of 1:10 (e.g., 0.4 mL into 3.6 mL) in neutralizing Letheen broth. This procedure provided theoretical quantitation of a 4 log unit decrease and detection of a 5 log unit decrease in cfu/mL. The PKCA-EP and EP control solutions were spiral plated with 50 µL of each test sample onto the appropriate agar as applicable to attain countable dilutions. In addition, each of the neutralized tubes of test sample and agar plates were incubated overnight in an appropriate atmosphere and temperature. After determining the bacterial counts, the test sample tubes where the bacteria had been exposed to EP were incubated for another 24 hours. If no bacterial suspension was observed in those tubes, then it was an indication that a complete decontamination had occurred. The plate count results are illustrated in the Table below.

| | Log Reduction |
|---|---|
| Control | 5.9 |
| PKCA Compound | 4.9 |
| 2% Ethyl Pyruvate | 4.9 |

As data in the above Table shows, a 2% concentration of ethyl pyruvate in the PKCA solution did not inhibit the biocidal efficacy of the PKCA compound. The surprising and unexpected result was that ethyl pyruvate also killed 4.9 log units of MRSA itself.

*Burkholderia pseudomallei* (*B. pseudomallei*) and *Burkholderia mallei* (*B. mallei*) are the causative agents of melioidosis and glanders, respectively. These are gram-negative pathogens, and unlike MRSA (a gram positive bacteria), are endemic in many parts of the world. Without being bound by any theory, it is believed that these bacteria are resistant to many, if not most, antibiotics because of their ability to pump (i.e., remove) the antibiotics out of their cell using an active transport system. Although natural acquisition of these pathogens is rare in the majority of countries, these bacteria have recently gained much interest because of their potential as bioterrorism agents.

In another study, ethyl pyruvate was tested to determine the effects of 2% ethyl pyruvate on *B. pseudomallei.* For this study, *B. pseudomallei* 1026b was inoculated into 3 mL of Letheen broth and incubated overnight at 37 °C. The next day 20 µL of the overnight culture as added to 2 mL of Letheen broth with 0.1% sodium thiosulfate to achieve ∼10⁷ cfu/mL. Afterwards, this solution was diluted to a working solution of ∼10⁴ cfu/mL. Two test tubes with 5 mL of 2% ethyl pyruvate were prepared and two tubes with 5 mL sterile water were prepared as controls. A 100 µL of ∼10⁴ cfu/mL stock solution of *B. pseudomallei* 1026b was added directly to one positive control tube containing 5 mL of sterile water, and 100 µL of ∼10⁴ cfu/mL stock solution of *B. pseudomallei* 1026b was added directly to one tube containing 5 mL of 2% ethyl pyruvate. These tubes were incubated at 37 °C for 20-24 hours and then observed for growth (+) or no growth (-). After 24 hours of incubation, there was no noticeable growth in the *B. pseudomallei* 1026b inoculated ethyl pyruvate tube and a significant growth in the negative control. This indicates that ethyl pyruvate has antibacterial activity even against bacteria that are highly resistant to broad spectrum antibiotics.

Although unlikely, the potential hydrolysis product ethanol was considered as a possible source of ethyl pyruvate's antimicrobial activity. To determine whether ethanol was the source of antimicrobial activity, analytical experiments were performed to determine if the hydrolysis occurred over time to produce ethanol.

Fourier transform infrared (i.e., FTIR) scans of 30% ethyl pyruvate in 50% hydrogen peroxide solution incubated at room temperature for 1 hour, 16 hours and 60 days were obtained. Study of these FTIR scans showed that no ethanol was produced during incubation in hydrogen peroxide solution even after 60 days. This result indicates that ethyl pyruvate is stable in the PKCA solution since the concentration of the hydrogen peroxide in those solutions is approximately only half that of 50% hydrogen peroxide solution.

An equivalent mixture of ethyl pyruvate and hydrogen peroxide that was incubated at room temperature for 7 days was analyzed by FTIR and by gas chromatography. There was no ethanol found in the mixture. This result further indicates stability of ethyl pyruvate in the presence of hydrogen Peroxide.

Typically, a 70% ethanol solution is used disinfection. Therefore, it is highly unlikely that a concentration of 2% ethanol (if all of the ethyl pyruvate is hydrolyzed) would kill bacteria in a 10 minute time period. Without being bound by any theory, it is possible that esterase enzyme in bacteria may hydrolyze ethyl pyruvate to produce ethanol *in situ* resulting in the observed antibacterial activity. Another possibility is that the antimicrobial effect is due to the decrease in pH from the release of the pyruvic acid.

### EXAMPLE 13

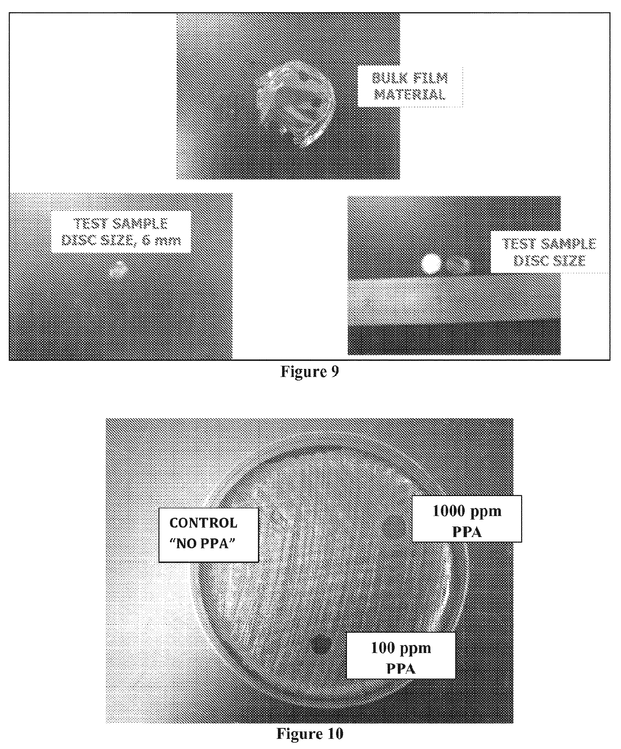

In this example, a composition of the invention was formulated in bandage materials or as dissolvable films (Figure 9) such that the active composition is released when moisture is present. Bandages and films can be formulated for sustained time release, thereby providing the composition of the invention to the wound over a prolonged period. Different bandage materials can be used, for example, they can be air permeable or substantially non-air permeable or sealed. In addition, bandages and films comprising a composition of the invention can be fabricated with other conventional bandaging materials and then stored in dry form for use, for example, for combat field use during evacuation and level 2-4 transports. Other possible formulations for compositions of the invention include, but are not limited to, gels, lotions, cream, or other suitable formulations that can be directly applied to wounds. In some embodiments, formulations of the composition of the invention enable an effective time release. Typically, such formulations are light weight and stable forms of wound dressing materials that can be applied directly to the wound. In some embodiments, compositions of the invention are formulated such that they are released when exposed to wound. Often such formulations dissolve over time releasing the composition of the invention. Such formulations have broad application to both the military and civilian population. For example, such formulations for military use include, but are not limited to, immediate field application upon initial triage through the entire course of the wound healing process.

Figure 10 shows the result of treating MRSA on blood agar plate with a composition of the invention comprising PPA that was incorporated in dissolvable film (see Figure 9). As the results show, the control film disc was completely grown over while the dissolvable film comprising a composition of the invention killed *MRSA* relatively in proportion to the PPA concentration.

The foregoing discussion of the invention has been presented for purposes of illustration and description. The foregoing is not intended to limit the invention to the form or forms disclosed herein. Although the description of the invention has included description of one or more embodiments and certain variations and modifications, other variations and modifications are within the scope of the invention, e.g., as may be within the skill and knowledge of those in the art, after understanding the present disclosure. It is intended to obtain rights which include alternative embodiments to the extent permitted, including alternate, interchangeable and/or equivalent structures, functions, ranges or steps to those claimed, whether or not such alternate, interchangeable and/or equivalent structures, functions, ranges or steps are disclosed herein, and without intending to publicly dedicate any patentable subject matter.

## Claims

1. A composition comprising a peroxy α-ketocarboxylic acid, which contains an acidic -OOH group, and at least one of a carboxylic acid corresponding to the peroxy α-ketocarboxylic acid and an anion of the carboxylic acid corresponding to the peroxy α-ketocarboxylic acid, wherein the acidic -OOH group of the α-ketocarboxylic acid is replaced by a -OH group in the carboxylic acid corresponding to the peroxy α-ketocarboxylic acid.

2. The composition of claim 1 further comprising the corresponding decarboxylated carboxylic acid of the peroxy α-ketocarboxylic acid, which contains a carbonyl carbon with a double bond to oxygen and a single bond to an acidic -OOH group and an α-carbon bound to the carbonyl carbon, wherein the corresponding decarboxylated carboxylic acid of the peroxy α-ketocarboxylic acid lacks the carbonyl carbon of the peroxy α-ketocarboxylic acid and a carbon corresponding to the α-carbon in the peroxy α-ketocarboxylic acid includes a double bond to oxygen and a single bond to -OH.

3. The composition of claim 2 further comprising hydrogen peroxide.

4. The composition of any one of claims 1 to 3, further comprising at least one of metabolic growth factors, antibiotics, antimycotics, and stimulators.

5. The composition of one of claims 1 to 4 formulated as a cream, gel, ointment, lotion, patch.

6. The composition of one of claims 1 to 4, further comprising an aqueous or oily base and at least one of a thickening or gelling agents such that the composition is formulated as an ointment or cream.

7. The composition of one of claims 1 to 4, further comprising an aqueous or oily base, and at least one of an emulsifying agent, a stabilizing agent, a dispersing agent, a suspending agent, a thickening agent, or a coloring agent such that the composition is formulated as a lotion.

8. The composition of one of claims 1 to 4, wherein the composition is micronized.

9. The composition of one of claims 1 to 4, wherein the composition is an aerosol with a particle size of 5 µm or less.

10. The composition of claim 9, further comprising a chlorofluorocarbon (CFC).

11. The composition of claim 10, wherein the chlorofluorocarbon is selected from dichlorodifluoromethane, trichlorofluoromethane, or dichlorotetrafluoroethane.

12. The composition of any one of claims 9 to 11, further comprising a surfactant.

13. The composition of claim 12, wherein the surfactant is lecithin.

14. The composition of one of claims 1 to 4, further comprising carbon dioxide gas.

15. The composition of claim 14, further comprising a surfactant.
